# EUROPEAN PATENT SPECIFICATION

(11) **EP 1 032 915 B1**
(45) Date of publication and mention of the grant of the patent: **09.05.2012**
(21) Application number: 98960549.8
(22) Date of filing: 25.11.1998
(51) Int. Cl.: G06K 9/00, G06F 19/00

(54) **COMPUTER-AIDED DIAGNOSIS SYSTEM AND METHOD**
SYSTEM UND VERFAHREN ZUR RECHNERGESTÜTZTEN DIAGNOSE
SYSTEME ET PROCEDE DE DIAGNOSTIC ASSISTE PAR ORDINATEUR

(30) Priority: 28.11.1997 US 980254
(43) Date of publication of application: 06.09.2000
(73) Proprietor: Wang, Shih-Ping, Los Altos, California 94022 (US)
(72) Inventor: Wang, Shih-Ping, Los Altos, California 94022 (US)
(74) Representative: Cross, James Peter Archibald
(86) International application number: PCT/US1998/025357
(87) International publication number: WO 1999/028857

(56) References cited:
- EP-A- 0 638 874
- WO-A-95/14950
- WO-A-96/36014
- DE-A- 3 840 257
- US-A- 5 331 550
- US-A- 5 586 160
- US-A- 5 586 160
- US-A- 5 627 907
- US-A- 5 657 362
- US-A- 5 815 591

## Description

### Reference to Related Applications

This application is a continuation-in-part of copending parent applications Serial Nos. 08/579,802 filed on December 28, 1995 and 08/438,432 filed on May 10, 1995 (allowed). In turn, Serial No. 08/579,802 is a continuation, and Serial No. 08/438,432 is a continuation-in-part, of parent application Serial No. 08/129,255 filed on September 29, 1993 (abandoned). This application hereby incorporates by reference the entire disclosure, drawings and claims of each of said parent applications as though fully set forth herein.

### Field and Background of the Invention

This invention relates to displaying radiological images and other information in a manner which is believed to assist users such as physicians in reading such images and other information. More specifically, the invention relates to a computer-aided diagnosis ("CAD") system and method for detection and identification of abnormalities from radiological images which can be viewed in a conventional format but in conjunction with viewing an annotated road map of the location and/or the identification of suspected abnormalities found in accordance with the invention through computer processing of the conventionally obtained radiological image. The annotated map highlights and/or identifies suspected abnormalities to help the reader better assess the presence and/or meaning and significance of abnormalities in the conventionally obtained radiological image.

The detection of abnormal anatomic regions in radiological images using a computer system comprising specialized software and possibly specialized hardware has been reported. For example, in the area of mammography, representative reports are :WO 96/36014 Gager et al in the May 1993 issue of RadioGraphics, pages 647-656; Giger et al in Proceedings of SPIE, Volume 1445 (1991), pages 101-103; Doi et al in U.S. Patent No. 4,907,156; and Giger et al in U.S. Patent No. 5,133,020. See, also, the disclosure of and in prior art cited in said parent applications. In particular, in the area of detecting spiculated or stellate lesions in applications. In particular, in the area of detecting spiculated or stellate lesions in mammograms using convergent line detectors as the principal abnormal feature detection algorithm, representative reports are: N. Karssemeijer in the book entitled "Digital Mammography", edited by A. G. Gale et al, published by Elsevier in 1994, pages 211-219; and Kegelmeyer et al in Volume 191 (1994) of Radiology, pages 331-337. In the area of detecting clusters of microcalcifications in mammograms using thresholding and a clustering kernel as the principal abnormal feature detection algorithm, representative report are: Nishikawa et al in Volume 20 (1993) of Medical Physics, pages 1661-1666; and Feig et al in Volume 33 (1995) of Radiological Clinics of North America, pages 1205-30. See. also, copending patent applications Serial No. 08/676,660 filed on July 10, 1966 entitled "Method and apparatus for fast detection of spiculated lesions in digital mammograms," and Serial No. 08/901,541 filed on July 28, 1997 entitled "Method and system for using local attenuation in the detection of abnormalities in digitized medical images." These two patent applications and each of the other references cited in this patent specification are incorporated herein by reference as though fully set forth herein. These systems are generally referred to as Computer-Aided Diagnosis ("CAD") systems, and are believed to be particularly useful to radiologists in the diagnostic process and particularly in screening radiological procedures.

In a screening radiological procedure, such as screening mammography, the patients typically are asymptomatic and true abnormalities (e.g. cancers) are said to occur at a typical rate of about one case per one hundred patient examinations. Reading of the mammograms, when most of them are negative, can be a tedious task that can make it difficult to maintain a constantly high attention level. Some detectable abnormalities can be missed or misdiagnosed, which can result in delayed or more costly treatment, and can even result in a reduction of patient's longevity or chance of survival. According to an article in the May 26, 1993 issue of JAMA, pages 2616-2617, the misdiagnosis rate in mammograms can be in the range of 15 to 63%. The CAD system, serving as an electronic reminder or second reader, as a spell-checker can be in a word processor, can assist radiologists in attaining higher detection rate (higher sensitivity) for abnormalities or reducing the misdiagnosis rate (lowering the false-negative rate).

Applicant understands that a current procedure using a CAD mammographic system proceeds as follows. The physician views a radiological image, reaches a preliminary diagnostic decision, and then views a separate second image displayed on a CAD system. This second image is marked or annotated with a localized identification of the abnormalities that the CAD system has detected through computer analysis of a digitized version of the conventionally obtained radiological image. After a reexamination the area of the radiological image that corresponds to the position of the detected abnormalities displayed on the CAD system, the physician makes the final diagnostic decision. This final diagnostic decision may or may not be the same as the preliminary decision, depending on whether the physician found the additional diagnostic information provided by the CAD system to be significant and, if so, what significance the physician ascribed to it. Following the final diagnostic decision, and perhaps depending on the degree of suspicion for malignancy, the physician can recommend a course of further action, which can include no further action or further follow-up examinations or biopsy.

In the process of detecting abnormal anatomic features in radiological images using a CAD system as described in the above cited references, the radiological film image of a patient is processed through a film digitizer to generate a digitized image which is input as such into the system. The digitized image is then analyzed by a digital image processing computer with specialized software and perhaps also specialized hardware for abnormal anatomic feature detection. If abnormalities are detected, an annotated radiological image is displayed on a special TV monitor, with markers placed around or adjacent the detected abnormalities. This TV monitor typically has a large dimension (typically a screen diagonal of 12 inches or larger) and a high spatial resolution (typically more than 1000 x 1000 pixels). Because of the large dimension and high spatial resolution, this TV monitor typically is positioned at some distances away from the film. Typically the center of the monitor is more than 12 inches from the center of the film on the conventional film illumination box. In addition, this special TV monitor typically has a low brightness and a high cost.

While the above described CAD system can point out the CAD-detected abnormalities to the physician, it is believed that the display method that it utilizes, using a high-resolution TV monitor, has certain shortcomings which make the process of using it inconvenient and inefficient. The high-resolution TV monitor is expensive, its spatial resolution although high for monitors is still much less than that of the original x-ray film, and its brightness and dynamic range are also very much inferior to those of an x-ray film viewed on a light box. Therefore, it is believed that a physician would not rely solely on the image displayed on the TV monitor to make diagnosis, but typically would repeatedly go back to the conventional film illumination box to view the original film image. This can lead to the loss of valuable time and can be uncomfortable at least because of the different brightness levels and spatial resolution levels of the two images. In addition, it is believed that diagnostic errors can arise from the need for the physician to shuttle back and forth between two different displayed images. Even when a potentially true abnormality (cancer) is detected and pointed out by the CAD system to the physician, the fatigue and eye discomfort and other effects due to viewing two images of such different characteristics may still cause the physician to miss the significance of the corresponding area on the original x-ray film and to fail to notice or appreciate the abnormal features of the detected abnormality and decide to ignore the detected abnormality.

### Summary of the Invention

An object of the invention is to provide an improved combined display of an x-ray radiological image and CAD-detected abnormalities from the x-ray image. A more specific object is to provide the CAD user with further processed, annotated and enhanced image tiles of the regions around the CAD detected abnormalities for the purpose of emphasizing the abnormal image features of these detected abnormalities so that the user (physician) can better assess the type and degree of abnormality of these detected abnormalities in the radiological image.

Another objective is to present the further processed image of the area around the CAD detected abnormalities on a small TV monitor located in such close proximity to the x-ray film during viewing of the x-ray film that eye and other discomfort due to viewing two different images alternately would be reduced. Still another object of the invention is to print the annotated road map and/or the further processed image tiles of the area around the CAD detected abnormalities on the same sheet of photographic film that contains a printout of the radiological image.

For example, in the area of systems and methods for displaying processed and enhanced X-ray images using multiple display means representative publication is WO 97/19399.

In an exemplary and non-limiting first embodiment of the invention, the further processed image of the area around the CAD detected abnormalities from a radiological film is presented on a small TV monitor, located in close proximity to the radiological film being viewed at the light box. The display of this further processed image shares (e.g., is toggled on) the small TV monitor with the display of a miniaturized annotated road map. On demand by the CAD user, e.g. the physician using a toggle switch, the miniaturized annotated road map image and the further processed image tiles of the areas around the CAD detected abnormalities are displayed alternatively on the small TV monitor.

In another exemplary and non-limiting second embodiment of the invention, the miniaturized annotated road map image is presented on a small TV monitor and the further processed image tiles of the areas around the CAD detected abnormalities from a radiological film are presented on a second and separate small TV monitor, both located in close proximity to the radiological film being viewed at the light box.

In the case of a radiological image that has been acquired through digital means, and thus is in digital form initially, the final image is frequently printed on a sheet of photographic film for later viewing on a light box. In an exemplary and non-limiting third embodiment of the invention, both a miniaturized annotation road map and the further processed image of the area around the CAD detected abnormalities from a radiological film are printed on the same sheet of photographic film that contains a print of the x-ray radiological image.

In an exemplary and non-limiting fourth embodiment of the invention, a particularly simple digital radiography system comprises a CAD system and an analog acquisition system.

In an exemplary and non-limiting fifth embodiment of the invention, a CAD system is used to decide how the relatively wide latitude digitally acquired image should be displayed on a display medium such as film having a narrower latitude.

### Brief Description of the Drawings

Figure 1 is a block diagram illustrating a CAD system and its output display according to a first embodiment of the invention.
Figure 2 is a block diagram illustrating a CAD system and its output display according to a second embodiment of the invention.
Figure 3 is a block diagram illustrating a CAD system and a first method of output display according to the third embodiment of the invention.
Figure 4 is a block diagram illustrating a CAD system and a second method of output display according to the third embodiment of the invention
Figure 5 is a block diagram illustrating a CAD system and an output display according to a fourth embodiment of the invention.
Figure 6 illustrates displaying an output according to a fifth embodiment of the invention.

### Detailed Description

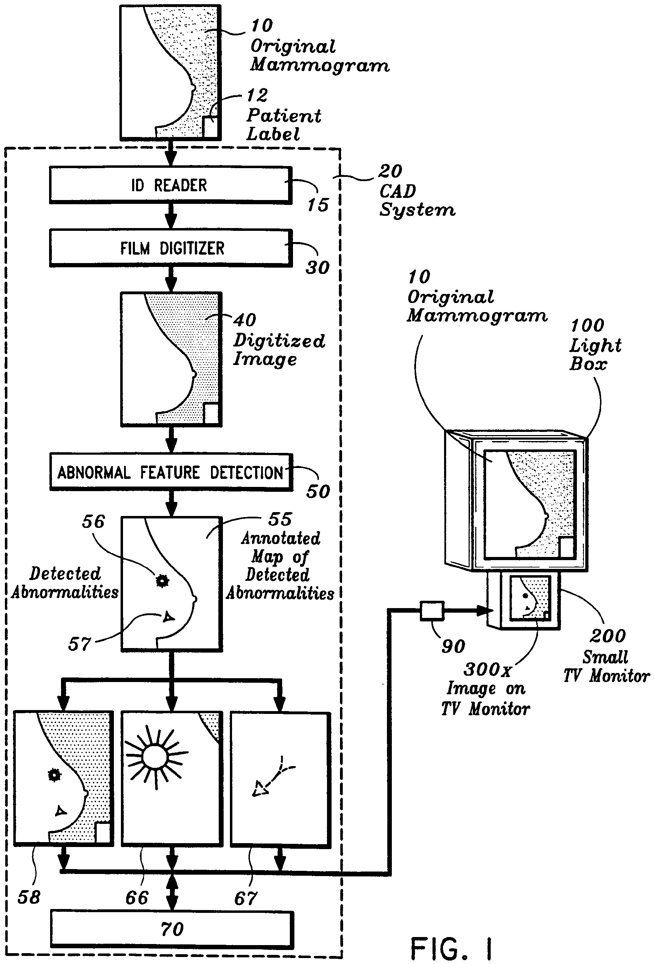

Referring to Figure 1, a preferred but non-limiting example, according to a first embodiment of the invention, generates an annotated road map of CAD-detected abnormality and a further processed image of the area around the CAD detected abnormalities from a radiological film. Both the annotated road map and the further processed image are displayed on a small TV monitor located in close proximity to the radiological film being viewed at a light box. In this example, the radiological film is in the form of a mammographic x-ray film, which is acquired with a conventional mammographic film-screen imaging system. The original analog two-dimensional mammographic x-ray film **10,** with a patient information label **12** printed on the edge of the film, is sent through a film digitizer **30** of a CAD (computer-aided diagnosis) system **20** (such as that disclosed in said U.S. patent applications which are incorporated by reference herein) to obtain a digitized two-dimensional mammographic image **40**. Preferably, the film digitizer **30** should be a laser film digitizer or a high performance CCD based film digitizer and should have a dynamic range and a spatial resolution comparable to those of the original mammographic film which typically has a dynamic range of 10,000:1 and spatial resolution of approximately 50 microns per pixel (or about 4,000 x 5,000 pixels for a 8-inch x 10-inch film). The identity of the original mammographic image **10** is entered into the CAD system at this point to identify the digitized mammographic image **40** and thus the original film **10**. An useful option at this point is to automatically input the identity of this original mammographic image **10** into the CAD machine. This can be accomplished, for example, by labeling the mammographic film **10** with a code such as a bar code next to the a patient information label **12** printed on the edge of the film, or by incorporating the bar code into the patient information label 12. and then reading the label into the CAD system **20** with an optional ID bar code reader **15** as the mammographic film **10** is being fed into the film digitizer **30**.

The digitized mammographic image **40** is then sent through an abnormal feature detection stage **50** of the CAD system, or CAD machine, **20.** The findings or results, positive or negative in nature, from the abnormal feature detection stage **50** are in the form of a two-dimensional annotation map **55**, or x-y coordinate information, of the locations and types of the CAD-detected abnormalities **56** and **57** (in this illustrative example) present in the original film image **10**. For the purpose of illustration, let the abnormality **56** be a spiculated lesion and let its location on the annotated map be marked with a star-shaped marker. Let the abnormality **57** be a cluster of microcalcifications and let its location on the annotated map be marked with a triangular shaped marker. Thus, the markers identify not only the detected location but also the detected nature of the suspected abnormality identified at this stage. The annotation map **55** can be scaled down to the same size of a sub-sampled image, say 512 x 512 pixel in size and 8-bit in gray scale, of the digitized image **40**, and the two superimposed images in registration with each other forms a miniaturized annotated road map image **58**. Enhanced image tiles **66** and **67**, centered respectively around the CAD-detected abnormalities **56** and **57**, say 512 x 512 pixel in size and 8-bit in gray scale, are generated by further image processing the regions in the digitized image 40 which correspond to the CAD detected abnormalities **56** and **57.** The CAD-generated annotation map **55**, the miniaturized annotated road map image **58,** the enhanced image tiles **66** and **67**, together with the digitized image **40** and its corresponding identification, may be stored for later use in an optional memory storage unit **70**.

The annotation road map **58** and the enhanced image tiles **66** and **67** are transferred to an output display section of the system for display. The output display section of the CAD system can be a part of the total CAD system, and in which case the data transfer is conducted through a dedicated shielded cable. Or, the output display section can be a separate system, in which case an additional data storage memory can be added to the unit to store the transferred interim data and the data transfer can be through a dedicated shielded cable or an existing network where the equipment is installed.

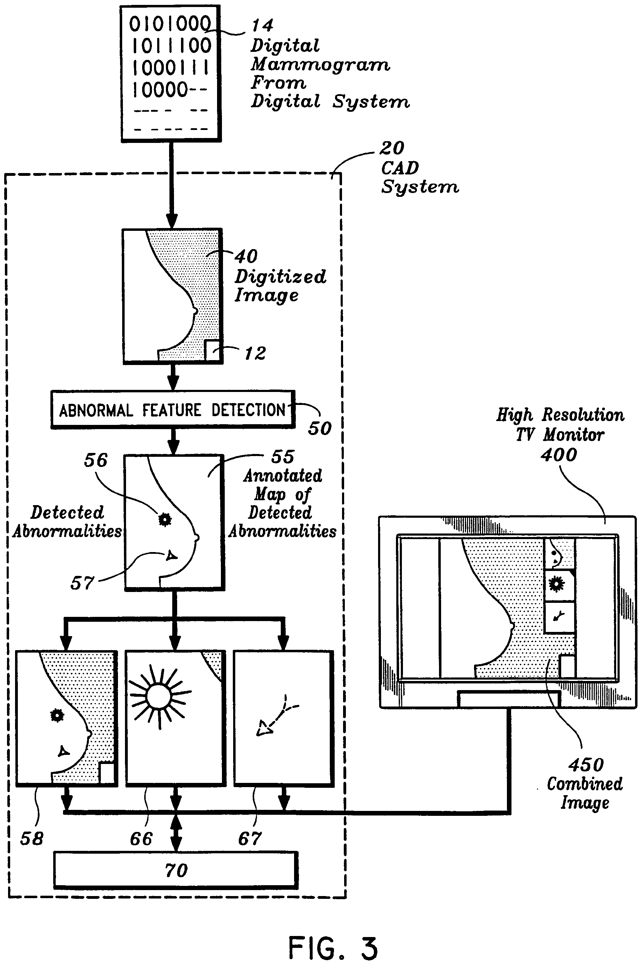

It is important to point out and emphasize the abnormal features of the CAD detected abnormalities to the physician, because it is believed that the physician, even after seeing the location of the CAD detected abnormalities on the miniature road map 58, can fail to notice or appreciate these abnormal features on the original the x-ray film. By pointing these abnormal features out, with further emphasis, to the physician, it is believed that the physician would be in better position to assess the level of abnormality of these CAD detected abnormalities. The principal abnormal feature detection algorithms used in the abnormal feature detection stage **50** to detect the abnormalities can be used to further emphasize the abnormal features of the CAD detected abnormalities. For example, in the case of the abnormality **56**, the principal abnormal feature used to detect the spiculated lesion can be a set of convergent lines. Since the presence of the convergent lines around a lesion raises the probability of malignancy, it is believed that there would be less a chance that the physician could ignore the lesion if the convergent lines were made more noticeable. Therefore, this set of convergent lines around the spiculated lesion could be contrast and edge enhanced to form the image tile **66**. For example, Figure 3(B) of Karssemeijer article cited shows a set of detected pixels pointing to the center of a suspected spiculated lesion. Superimposing these detected pixels on the image can form the image tile 66. In the case of the abnormality **57**, the principal abnormal feature used to detect the cluster of microcalcifications would be the small clustering of three or more high contrast spots. This small clustering of high contrast spots could be enhanced in brightness to form the image tile **67**. The formation of this small clustering of bright spots can be of great interest to the physician. This is because the probability of malignancy is higher for a linear or branching formation. Therefore, this small clustering of high contrast spots should also be magnified in size, for example by a factor of 2 or more, to form the image tile **67** in order to help the physician see the formation of these bright spots clearly in the image tile **67**. In this manner, it is believed that the CAD user, the physician, after seeing the enhanced abnormal image features of these detected abnormalities and reexamining the original x-ray image, can better assess the level of abnormality of these detected abnormalities in the x-ray image.

Also shown in Figure 1 is an illustration of a CAD output display consisting of a conventional film illuminator, commonly called a light box, **100** and a small TV monitor **200** according to the first embodiment of the invention. In this exemplary embodiment, the miniaturized annotated road map **58** and the enhanced image tiles **66** and **67** are alternatively or sequentially displayed as images **300x**, by operating a toggle switch 90 to display one image at a time (where x = a, b and c) on the small TV monitor **200** located in close proximity to the original film **10**. Respectively, the image **300a** represents the miniaturized annotated road map **58**, the image **300b** represents the enhanced image tile **66** around the CAD detected spiculated lesion, and the image **300c** represents the enhanced image tile **67** around the CAD detected cluster of microcalcifications. If more abnormalities are detected, there would be images **300d**, **300e**, etc. to be toggled through the small TV monitor.

The dimension of the display screen of the small TV monitor **200** in this example of the invention are of the order of 1/4 to ½ of the dimension of original film **10**. In addition, the small TV monitor **200** should be located as close as practical to the light box **100** displaying the original film **10**. Preferably the center of the small TV monitor **200** should be less than 12 inches from the center of the original film **10** on the conventional film illumination light box **100**. The preferred position, as shown in Figure 1, for mounting the small TV monitor **200** is just beneath the light box **100** which displays the original image **10**. It is also convenient to display a pair of images on each TV monitor, since frequently a pair of the original mammographic films **10**, such as the mammograms of the left and right breasts, are displayed and viewed next to each other. In this manner, the physician still has to minimally move his or her eyes back and forth between the original radiologic film image **10** on the film illuminator **100** and the images **300x** displayed on the small TV monitor **200**. The spatial resolution of the small TV monitor **200** can be in the range of 500 TV lines, or comparable to that of NTSC or PAL. The brightness level of the small TV monitor **200** should be similar to that of the average brightness transmitted through the original film **10**, so that the observer would not be bothered by a change in brightness. In using the CAD system as a second reader in a screening situation, it is sometimes preferred that the display on the small TV monitor **200** can be easily toggled with on-off with a switch **90** by the observer.

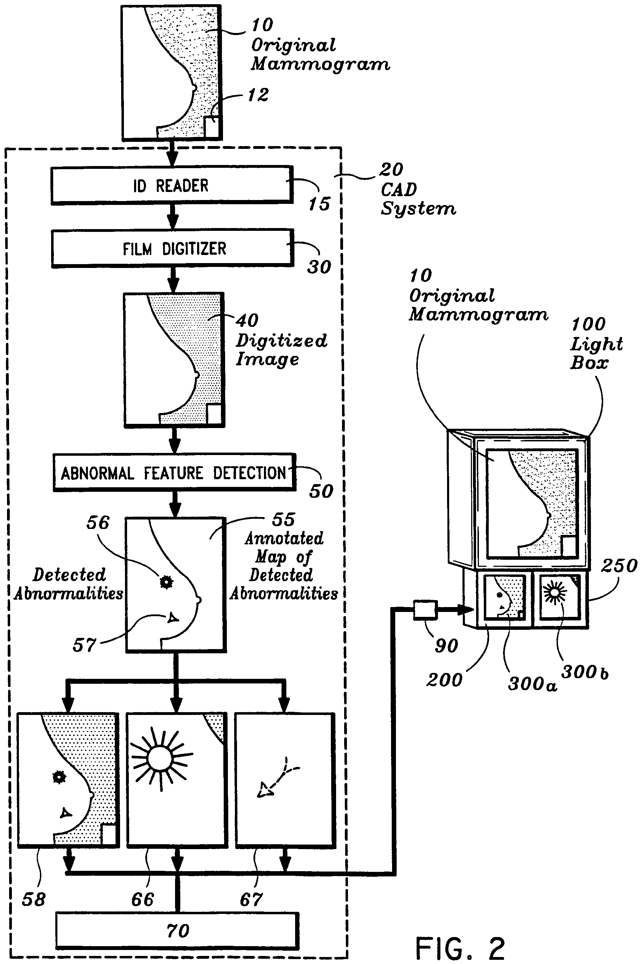

Figure 2 is similar to Figure 1 in many respects, and similarly labeled components serve a similar function and therefore will not be described again in detail. Figure 2 shows a CAD output display comprising a conventional film illuminator **100** and, in this case, two small TV monitors **200** and **250**, according to the second embodiment of the invention. In this exemplary embodiment, the annotated information **58** is presented as a miniaturized annotated road map image **300a** on the first small TV monitor **200**, located in close proximity to the original film **10**. The enhanced image tiles **66** and **67** are alternatively or sequentially presented, by operating a toggle switch **90**, as images **300b** and **300c** on the second small TV monitor **250**, located next to the first small TV monitor **200** and in close proximity to the original film **10**. It is sometimes preferred that two or more small TV monitors are used, in place of monitor **250**, to display the further processed image tiles 66 and 67 such that each detected abnormality is displayed on a separate small TV monitor at the same time. The small TV monitors 200 and 250 can be placed at other positions relative to the light box **100**, e.g. to the side or above light box **100**.

Referring to Figure 3, a preferred but non-limiting example according to the third embodiment of the invention receives radiological images which already are in the digital format, detects abnormalities on these radiological images with a CAD system, and prints out these radiological images together with CAD results on photographic film. Again, components labeled the same as in Figures 1 and 2 serve similar functions and therefore will not be described again in detail. Digital imaging systems, such as magnetic resonance imaging ("MRI") systems, computed tomography ("CT") systems, ultrasound imaging systems, scintillation cameras, computed radiography ("CR") systems (such as Fuji's CR system based on stimulated emission phosphor detector), and recently reported digital radiography and digital mammography systems (for example, see Feig article cited earlier; using CCDs or amorphous silicon array detectors), provide radiological images in the digital format. In this example, the radiological image is in the form of a digital mammogram, which is acquired with a digital mammography system. This digital mammogram **14**, already having properly encoded identification and patient information **12**, is reformatted into the digitized mammographic image **40** and is sent through the abnormal feature detection stage **50** of the CAD machine **20**. If the information is already properly formatted for the CAD machine **20**. it is sent directly to and through the abnormal feature detection stage **50** of the CAD machine **20** without reformatting. The initial film digitization step used in the first and second embodiments for analog mammograms is not needed in this case. As in the first and second embodiments, the findings or results, positive or negative in nature, from the abnormal feature detection stage **50** are in the form of a two-dimensional annotation map **55**, or x-y coordinate information, of the locations and types of the CAD-detected abnormalities **56** and **57** from the original film image **10**. For the purpose of illustration, let the abnormality **56** be a spiculated lesion and let its location on the annotated map be marked by a star shaped marker. Let the abnormality **57** be a cluster of microcalcifications and let its location on the annotated map be marked by a triangular shaped marker. The annotation map **55** can be scaled down to the same size of a sub-sampled image, say 512 x 512 pixel in size and 8-bit in gray scale, of the digitized image **40**, and the two superimposed images in registration with each other form a miniaturized annotated road map image **58**. Enhanced image tiles **66** and **67**, centered respectively around the CAD-detected abnormalities **56** and **57**, say 512 x 512 pixel in size and 8-bit in gray scale, are generated by further image processing the regions in the digitized image **40** which correspond to the CAD detected abnormalities **56** and **57**. The CAD-generated annotation map **55**, the miniaturized annotated road map image **58**, the enhanced image tiles **66** and **67**, and together with the digitized image **40** and its corresponding identification, can be stored for later use in an optional memory storage unit **70**.

The annotation road map **58** and the enhanced image tiles 66 and 67 are transferred to the output display section of the system for display. There are several methods to display the CAD results and the digitally acquired mammogram. Since the digital system produces no film to start with at the acquisition, the first method is a totally filmless display by using a high resolution TV monitor **400**. The resolution should be at least 1000 x 1000 pixels. In this method the annotation road map **58**, the enhanced image tiles **66** and **67**, and the digital mammogram **40** are all displayed on the same TV monitor as a combined digital image **450** as shown in Figure 3. The annotation road map **58** and the enhanced image tiles **66** and **67** are shown placed at the edge or margin of the combined digital image **450.** Patient information **12** can also be displayed at the same edge or margin of the combined digital image **450.** The annotation road map **58** may alternatively be displayed by overlaying it on top of the digital mammogram **40.** This overlay may be toggled (switch not show) on and off so that the digital mammogram **40** can be examined without obstruction. The enhanced image tiles **66** and **67** can also be toggled (switch not shown) on and off.

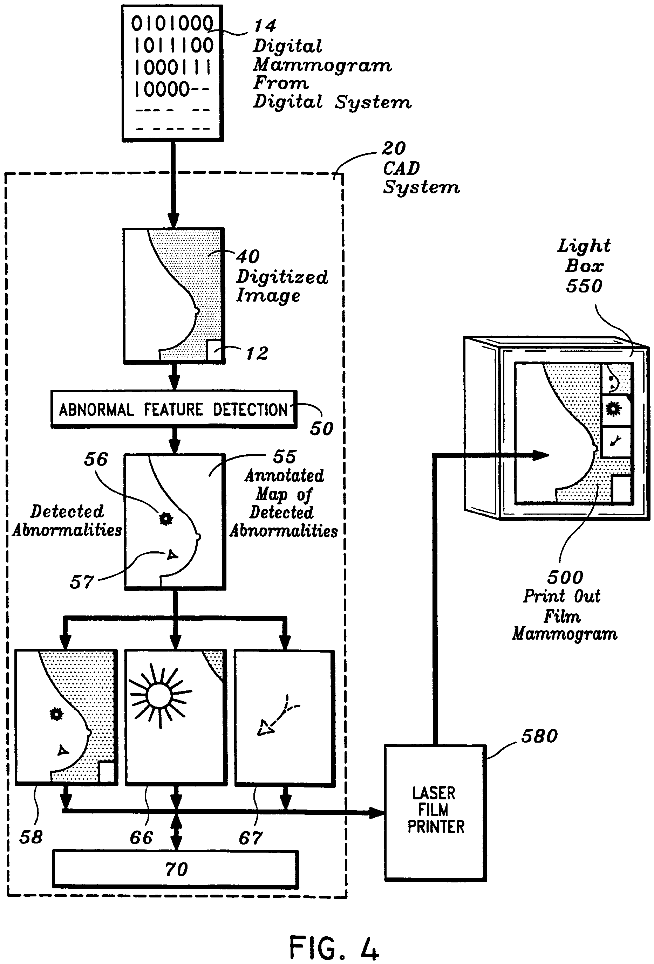

The second method of display, shown in Figure 4, where the same reference numerals have the same significance as in the earlier Figures, makes a photographic film printout of the digital mammogram **40**, the miniaturized annotation road map **58** and the enhanced image tiles **66** and **67** all on a same sheet of film 500. The printout film **500** is viewed on a light box **550**. Since, at the present time, physicians usually are more accustomed to a photographic film, which conveys information with much higher resolution and gray scale than a high resolution TV monitor, the second method of display can be preferred over the first method of display. The annotation road map **58** and the enhanced image tiles **66** and **67** are shown in Figure 4 placed at the same edge or margin of the printout film as the patient information label **12**. The photographic film printout, typically having a resolution of 4000 x 5000 pixels, can be made with a high resolution laser film printer **580**. Such high resolution, 4000 x 5000 pixels, laser film printers are commercially available with a resolution of 40 microns per pixel for 8 inch x 10 inch size films and 100 microns per pixel for 14 inch x 17 inch size films. It is sometimes preferred that only the miniaturized annotation road map 58 be printed at the edge of the printout film **500**.

Digital radiological images, such as in the case of images from magnetic resonance imaging ("MRI"), computed tomography ("CT"), digital fluorography ("DF"), and computed radiography ("CR"), are sometimes printed out on sheets of 4000 x 5000 pixels photographic film for later viewing on a light box. Since MRI images are typically formatted into 256 x 256 pixels, CT images into 512 x 512 pixels, DF images into 1024 x 1034 pixels, and CR images into 2048 x 2048 pixels, many images from MRI or CT or CR modalities can be printed as small tiles in an array on one sheet of 4000 x 5000 pixels photographic film. Therefore, the CAD findings from these images can be printed as one or several of the tiles. A further refinement on the use of CAD is to reduce the number of images to be presented to the physician, for example by not presenting radiological images on which no suspected abnormalities are found by the CAD system, or by not presenting such radiological images for a second opinion or review by another professional.

Although the embodiments discussed above have been described in terms of preferred structures involving a single sheet of film, it should be apparent to those skilled in the art that this invention applies to viewing of multiple films on a multiple-film viewing station or an alternator (a multiviewer having pre-loaded films and a transport belt), to allow several x-ray films **10** or printout films **500** to be viewed at the same time or different times, with or without their respective annotation maps **58** and the enhanced image tiles **66** and **67**.

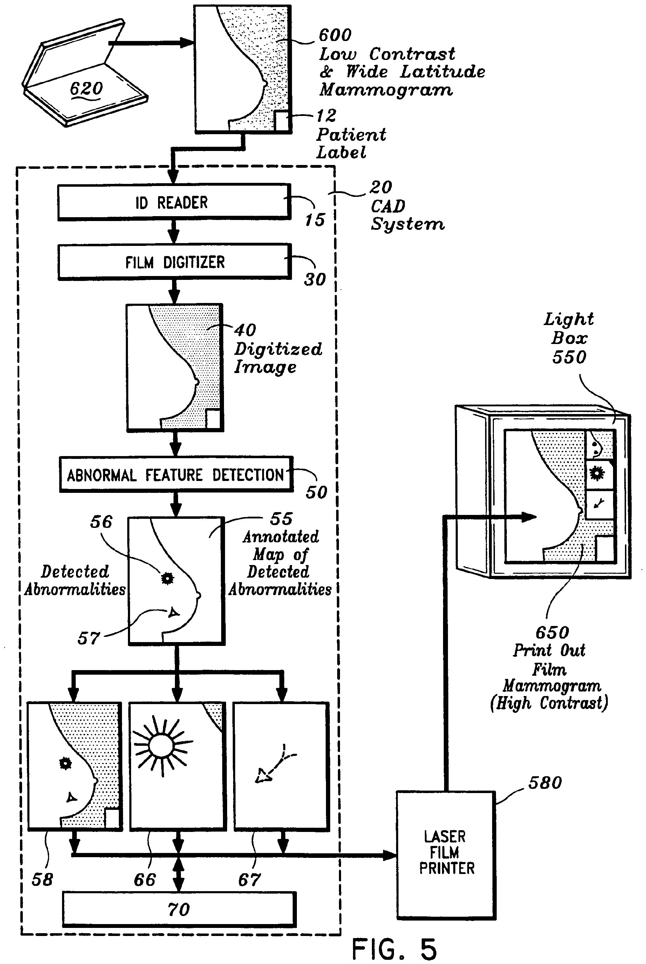

Figure 5 illustrates a particularly efficient digital radiography system according to the fourth embodiment of the invention. Again, the same reference numerals have the same meanings as in the earlier Figures. The Figure 5 system comprises an analog film-screen acquisition system and a CAD system similar to the third embodiment. In this example, the radiological image is in form of a low contrast and wide latitude mammographic film **600**. This embodiment and the third embodiment differ in the source of the digitized image **40**. The acquisition system, in this case, is an inexpensive conventional mammographic intensifying screen cassette **620**. The cost ratio between this screen cassette **620** and a typical current digital mammographic system is several order of magnitude, e.g., approximately 1000 times. The standard screen-film acquisition and exposure technique and the conventional mammographic x-ray system (not shown) will be unchanged for use in this embodiment of the invention. By reducing the contrast gradient G of the mammographic film **600** say from about 3.0 to about 2.0 to 1.5, we obtain a film with a factor of 5 to 25 more in latitude. That is, the exposure range expands from about 25 to about 125 to 630. The contrast gradient G is generally defined as the slope of the H & D [or film characteristics] curve where the optical density [or the log (base 10) of the reciprocal of the film transmission, the y-axis] is plotted against the log (base 10) of x-ray exposure [or light exposure. the x-axis]. Because of the low contrast, the physician cannot be expected to make diagnosis on this low contrast and wide latitude mammogram **600**. However, this mammographic film **600** can be digitized through the film digitizer **30** and reformatted into a digitized image **40**, and can be put through the CAD abnormal feature detection stage **50**. The reformatted version of the wide latitude image **600** is fed through a high resolution film printer **580** and printed out as a conventional high contrast mammogram **650**. Its respective annotated road maps **58** and the enhanced image tiles **66** and **67** are also printed at the edge or margin of the mammogram **650**. The physician, as in the third embodiment, finally reads the mammogram on the light box **550**. Using the miniaturized road map **58** as a guide, the physician reexamines the mammogram **650** to see if any of the CAD detected abnormalities suggest further action. The enhanced image tiles **66** and **67** provide the physician with further information by emphasizing the abnormal features of the CAD detected abnormalities. It is sometimes preferred that only the miniaturized annotation road map **58** be printed at the margin of the mammogram **650**.

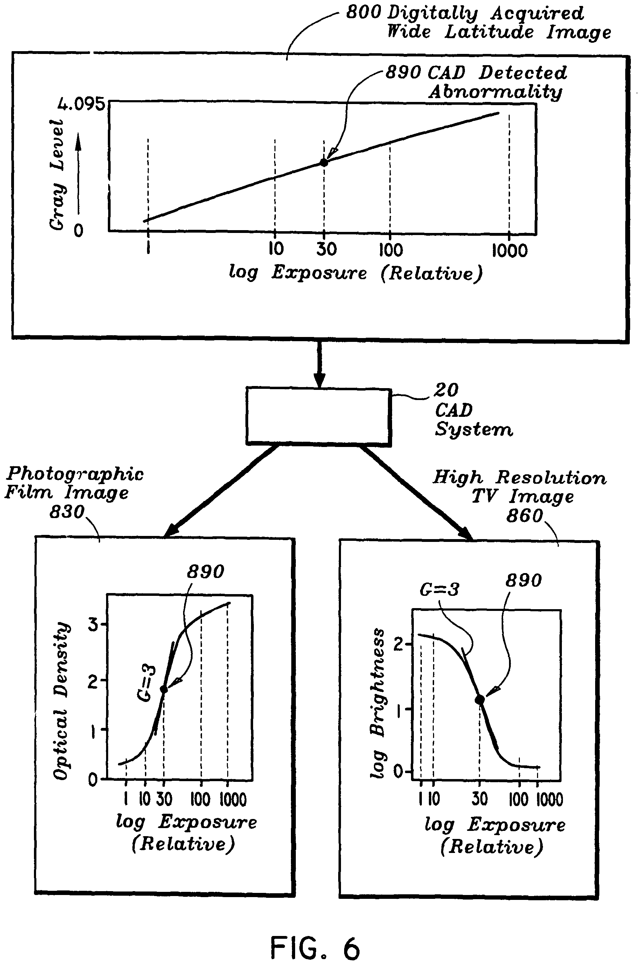

Figure 6 illustrates how a CAD system in accordance with a fifth embodiment of the invention can be used to decide how a wide latitude digitally acquired image should be displayed on a display medium having a narrower latitude. One of the major problems in digital imaging is how to display all the information contained in the digitally acquired images. Typically, according to Feig et al in Volume 33 (1995) of Radiologic Clinics of North America, pages 1205-30, and other sources, the exposure range of the latitude (the ratio of the exposure at the highest signal region to that at the lowest signal region) of the digitally acquired image is of the order of 100 to over 1000, which is much broader than that of the display media. As a comparison, if a radiogram is to be displayed on a photographic film, the exposure range of the latitude (the ration of exposure at the highest signal region to that at the lowest signal region where the display gradient is significant) is of the order of 25 and the exposure range of the latitude of a TV monitor is less than 10. As illustrated in Figure 6, a CAD system **20** is used to guide into two different display media the display of a wide latitude digitally acquired image **800** formatted for example such that the log of its output signal (with an exposure range of over 1000) is encoded into 12 bits (4096 levels of gray). In this example, the CAD system decides how the digitally acquired wide latitude radiogram **800** should be printed on a photographic film **830** (having a display latitude of, for example, 25) and displayed on a high resolution TV or computer monitor **860** (having a display latitude of, for example. 10). As illustrated in Figure 6, this is done by centering the display latitude of the display medium (photographic film **830** or monitor **860**) around the region of the CAD detected abnormality identified at **890** on the wide latitude digitally acquired image **800**. That is, only the range of say the relative exposure 10 to 100, around the CAD detected abnormality **890**, say around relative exposure 30, is provided at the optimum contrast gradient (say G=3.0) while other image content, above or below the exposure range of the CAD detected abnormality 890, are compressed in display latitude and are displayed with reduced contrast gradient on film **830** and/or monitor **860**.

Although the invention has been described in terms of preferred structures, methods and processes, it should be apparent to those skilled in the art that various alterations and modifications can be made without departing from the invention and that such modifications and alterations are intended to be considered to be within the spirit and scope of the invention as defined by the appended claims.

## Claims

1. A system comprising:
a computer-aided diagnosis system (20) arranged to process digital mammogram data (40) corresponding to a mammographic image to identify suspected spiculated lesion abnormalities (56) and microcalcification cluster abnormalities (57) by type and location and to produce annotated digital map data (55) corresponding to the mammographic image with symbols identifying the type and location of the abnormalities superimposed, and to process digital mammogram data for at least one of said abnormalities (56,57) to generate image data for at least one enhanced image tile (66,67), each enhanced image tile (66,67) comprising a display-enhanced version of a respective one of said abnormalities (56,57);
first display means (200;400;550) coupled to the computer-aided diagnosis system (20) for displaying an annotated digital map corresponding to the annotated digital map data;
second display means (100;400;550) for displaying a mammographic image (10;450) corresponding to the digital mammogram data (40); and
third display means (250;400;550) for displaying at least one enhanced image tile (66,67), wherein said at least one enhanced image tile comprises (i) a contrast- and edge-enhanced set of detected convergent lines if said abnormality is a spiculated lesion, and (ii) a brightness-enhanced set of detected cluster pixels if said abnormality is a microcalcification cluster, and
wherein the first display means and the third display means are arranged to display said annotated digital map (55) and at least one of the enhanced image tiles (66,67) concurrently with and adjacent to the mammographic image (10,450) as displayed by the second display means.

2. The system of claim 1, further including:
a source of a film mammogram (10); and
a film reader / digitizer (30) for converting the film mammogram (10) to said digital mammogram data (40); and
said first display means coupled to the computer comprises an electronic monitor (400).

3. The system of claim 1, wherein said at least one enhanced image tile further comprises a magnified version of said abnormality.

4. The system of claim 1, further including a mammography unit for supplying said digital mammogram data, and wherein said first, second, and third display means are integrated into a common electronic monitor (400) displaying a combined image (450) of said annotated digital map (55), said at least one enhanced image tile (66,67), and said mammographic image (10).

5. The system of claim 1, further including a mammography unit, and wherein said first, second, and third display means are commonly integrated into a film printer (580) and a light box (550), the film printer (580) printing said annotated digital map (55), said at least one enhanced image tile (66,67), and said mammographic image onto a common film print out (500), the light box (550) being operable to display the film print out (500).

6. The system of claim 1, further including:
an intensifying screen cassette mammography unit (620) for producing a low contrast, wide latitude film mammogram (600); and
a film reader/digitizer (30) for converting the low contrast, wide latitude film mammogram (600) to said digital mammographic data (40);
and wherein said first, second, and third display means are commonly integrated into a film printer (580) and a light box (550), the film printer (580) printing said annotated digital map (55), said at least one enhanced image tile (66,67), and said mammographic image (10,14,40) onto a common film print out (650), the light box (550) displaying the film print out (650).

7. The system of claim 1, wherein said first and second display means are mutually configured such that the annotated digital map (55) is displayed below the mammographic image (10).

## Patentansprüche

1. System, umfassend:
ein computergestütztes Diagnosesystem (20), das zur Verarbeitung digitaler Mammogrammdaten (40) ausgebildet ist, die einem mammografischen Bild entsprechen, zur Identifizierung vermutlicher Abnormalitäten in Form einer spikulierten Läsion (56) und Abnormalitäten in Form von Mikrokalzifizierungs-Clustern (57) nach Art und Position, sowie zum Erhalten kommentierter digitaler Kartendaten (55), die dem mammografischen Bild entsprechen, mit Symbolen, die die Art und Position der darüber gelegten Abnormalitäten identifizieren, und zum Verarbeiten digitaler Mammogrammdaten für mindestens eine der Abnormalitäten (56, 57), um Bilddaten für mindestens eine verstärkte Bildkachel (66, 67) zu generieren, wobei jede verstärkte Bildkachel (66, 67) eine anzeigeverstärkte Version jeweils einer der Abnormalitäten (56, 57) umfasst;
ein erstes Anzeigemittel (200; 400; 550), das an das computergestützte Diagnosesystem (20) gekoppelt ist, um eine kommentierte digitale Karte anzuzeigen, die den kommentierten digitalen Kartendaten entspricht;
ein zweites Anzeigemittel (100; 400; 550) zum Anzeigen eines mammografischen Bildes (10; 450), das den digitalen Mammogrammdaten (40) entspricht; und
ein drittes Anzeigemittel (250; 400; 550) zum Anzeigen mindestens einer verstärkten Bildkachel (66, 67),
wobei die mindestens eine verstärkte Bildkachel (i) einen kontrast- und randverstärkten Satz von erfassten konvergierenden Linien umfasst, wenn die Abnormalität eine spikulierte Läsion ist, und (ii) einen in der Helligkeit verstärkten Satz von erfassten cluster-Pixeln umfasst, wenn die Abnormalität ein Mikrokalzifizierungs-Cluster ist, und
wobei das erste Anzeigemittel und das dritte Anzeigemittel zum Anzeigen der kommentierten digitalen Karte (55) und mindestens einer der verstärkten Bildkacheln (66, 67) gleichzeitig mit und neben dem mammografischen Bild (10, 450) ausgebildet sind, das von dem zweiten Anzeigemittel angezeigt wird.

2. System nach Anspruch 1, des Weiteren enthaltend:
eine Quelle eines Film-Mammogramms (10), und
eine Film-Lesevorrichtung/Digitalisierungsvorrichtung (30) zum Umwandeln des Film-Mammogramms (10) in die digitalen Mammogrammdaten (40); und
das erste Anzeigemittel, das an den Computer gekoppelt ist, umfasst einen elektronischen Monitor (400).

3. System nach Anspruch 1, wobei die mindestens eine verstärkte Bildkachel des Weiteren eine vergrößerte Version der Abnormalität umfasst.

4. System nach Anspruch 1, des Weiteren enthaltend eine Mammografieeinheit zum Zuleiten der digitalen Mammogrammdaten und wobei das erste, zweite und dritte Anzeigemittel in einem gemeinsamen elektronischen Monitor (400) integriert sind, der ein kombiniertes Bild (450) der kommentierten digitalen Karte (55), der mindestens einen verstärkten Bildkachel (66, 67) und des mammografischen Bildes (10) anzeigt.

5. System nach Anspruch 1, des Weiteren enthaltend eine mammografische Einheit und wobei das erste, zweite und dritte Anzeigemittel gemeinsam in einem Filmdrucker (580) und einer Leuchtplatte (550) integriert sind, wobei der Filmdrucker (580) die kommentierte digitale Karte (55), die mindestens eine verstärkte Bildkachel (66, 67) und das mammografische Bild auf einem gemeinsamen Filmausdruck (500) druckt, wobei die Leuchtplatte (550) funktionsfähig ist, den Filmausdruck (500) anzuzeigen.

6. System nach Anspruch 1, des Weiteren enthaltend:
eine Verstärkerfolienkassetten-Mammografieeinheit (620) zum Erzeugen eines kontrastarmen Film-Mammogramms (600) mit großem Belichtungswertebereich; und
eine Film-Lesevorrichtung/Digitalisierurigsvorrichtung (30) zum Umwandeln des kontrastarmen Film-Mammogramms (600) mit großem Belichtungswertebereich in die digitalen mammografischen Daten (40);
und wobei das erste, zweite und dritte Anzeigemittel gemeinsam in einem Filmdrucker (580) und einer Leuchtplatte (550) integriert sind, wobei der Filmdrucker (580) die kommentierte digitale Karte (55), die mindestens eine verstärkte Bildkachel (66, 67) und das mammografische Bild (10, 14, 40) auf einem gemeinsamen Filmausdruck (650) druckt und die Leuchtplatte (550) den Filmausdruck (650) anzeigt.

7. System nach Anspruch 1, wobei das erste und zweite Anzeigemittel wechselseitig so konfiguriert sind, dass die kommentierte digitale Karte (55) unter dem mammografischen Bild (10) angezeigt wird.

## Revendications

1. Système comprenant :
un système de diagnostic assisté par ordinateur (20) conçu pour traiter des données de mammographie numérique (40) correspondant à une image mammographique en vue d'identifier des anomalies supposées de lésion spiculée (56) et des anomalies d'agrégat de microcalcifications (57) par type et emplacement afin de produire des données de carte numérique annotée (55) correspondant à l'image mammographique comprenant des symboles identifiant le type et l'emplacement des anomalies superposées, et pour traiter des données de mammographie numérique pour au moins l'une desdites anomalies (56, 57) afin de générer des données d'image pour au moins une mosaïque d'images améliorée (66, 67), chaque mosaïque d'images améliorée (66, 67) comprenant une version à affichage amélioré d'une anomalie respective parmi lesdites anomalies (56, 57) ;
un premier moyen d'affichage (200 ; 400 ; 550) couplé au système de diagnostic assisté par ordinateur (20) pour afficher une carte numérique annotée correspondant aux données de carte numérique annotée ;
un deuxième moyen d'affichage (100 ; 400 ; 550) pour afficher une image mammographique (10 ; 450) correspondant aux données de mammographie numérique (40) ; et
un troisième moyen d'affichage (250 ; 400 ; 550) pour afficher au moins une mosaïque d'images améliorée (66, 67), ladite mosaïque d'images améliorée comprenant (i) un ensemble de lignes convergentes détectées de contraste et de bord améliorés si ladite anomalie est une lésion spiculée, et (ii) un ensemble de pixels d'agrégat détecté de luminosité améliorée si l'anomalie est un agrégat de microcalcifications, et
le premier moyen d'affichage et le troisième moyen d'affichage étant conçus pour afficher ladite carte numérique annotée (55) et au moins l'une des mosaïques d'image améliorées (66, 67) en même temps que l'image mammographique (10, 450) et de manière adjacente à ladite image telle qu'affichée par le deuxième moyen d'affichage.

2. Système conformément à la revendication 1 comprenant en outre :
une source d'une mammographie sur film (10) ; et
un lecteur/numériseur graphique de film (30) pour convertir la mammographie sur film (10) en lesdites données de mammographie numérique (40) ; et
ledit premier moyen d'affichage couplé à l'ordinateur comprend un moniteur électronique (400).

3. Système conformément à la revendication 1, dans lequel ladite mosaïque d'images améliorée comprend en outre une version agrandie de ladite anomalie.

4. Système conformément à la revendication 1 comprenant en outre une unité de mammographie pour la fourniture desdites données de mammographie numérique, et dans lequel lesdits premier, deuxième et troisième moyens d'affichage sont intégrés dans un moniteur électronique commun (400) affichant une image combinée (450) de ladite carte numérique annotée (55), ladite mosaïque d'images améliorée (66, 67), et ladite image de mammographie (10).

5. Système conformément à la revendication 1 comprenant en outre une unité de mammographie, et dans lequel les premier, deuxième, et troisième moyens d'affichage sont intégrés en commun dans une imprimante de film (580) et un négatoscope (550), l'imprimante de film (580) imprimant ladite carte numérique annotée (55), ladite mosaïque d'images améliorée (66, 67), et ladite image mammographique sur un imprimé de film commun (500), le négatoscope (550) étant apte à afficher l'imprimé de film (500).

6. Système conformément à la revendication 1 comprenant en outre :
une unité mammographique de cassette d'écran d'intensification (620) pour produire une mammographie sur film à faible contraste et de large latitude (600) ; et
un lecteur/numériseur graphique de film (30) pour convertir la mammographie sur film à faible contraste et de large latitude (600) en lesdites données de mammographie numérique ;
et dans lequel lesdits premier, deuxième et troisième moyens sont intégrés en commun dans une imprimante de film (580) et un négatoscope (550), l'imprimante de film (580) imprimant ladite carte numérique annotée (55), ladite mosaïque d'images améliorée (66, 67), et ladite image mammographique (10, 14, 40) sur un imprimé de film (650), le négatoscope (550) affichant l'imprimé de film (650).

7. Système conformément à la revendication 1, dans lequel lesdits premier et second moyens d'affichage sont mutuellement conçus de manière à ce que la carte numérique annotée (55) soit affichée au-dessous de l'image mammographique (10).
